# EUROPEAN PATENT SPECIFICATION

(11) **EP 3 714 897 B1**
(45) Date of publication and mention of the grant of the patent: **30.06.2021**
(21) Application number: 19165015.9
(22) Date of filing: 25.03.2019
(51) Int. Cl.: A61K 38/17, C12Q 1/02

(54) **ARTIFICIAL SPUTUM, METHOD OF PRODUCING AN ARTIFICIAL SPUTUM**
KÜNSTLICHES SPUTUM, VERFAHREN ZUR HERSTELLUNG EINES KÜNSTLICHEN SPUTUMS
EXPECTORATION ARTIFICIELLE, PROCÉDÉ DE PRODUCTION D'UNE EXPECTORATION ARTIFICIELLE

(43) Date of publication of application: 30.09.2020
(73) Proprietor: IMLred GmbH, 82131 Gauting (DE)
(72) Inventor: BEUTLER, Markus, 85276 Pfaffenhofen an der Ilm (DE); DELAMOTTE, Lubov, 81369 München (DE)
(74) Representative: Manitz Finsterwald Patent- und Rechtsanwaltspartnerschaft mbB

(56) References cited:
- US-A1- 2004 072 730
- US-A1- 2018 201 922
- D. D SRIRAMULU: "Microcolony formation: a novel biofilm model of Pseudomonas aeruginosa for the cystic fibrosis lung", JOURNAL OF MEDICAL MICROBIOLOGY, vol. 54, no. 7, 1 July 2005 (2005-07-01), pages 667-676, XP55619953, ISSN: 0022-2615, DOI: 10.1099/jmm.0.45969-0
- SEBASTIAN KIRCHNER ET AL: "Use of Artificial Sputum Medium to Test Antibiotic Efficacy Against Pseudomonas aeruginosa in Conditions More Relevant to the Cystic Fibrosis Lung", JOURNAL OF VISUALIZED EXPERIMENTS, no. 64, 5 June 2012 (2012-06-05), XP55619986, DOI: 10.3791/3857
- C. FUNG ET AL: "Gene expression of Pseudomonas aeruginosa in a mucin-containing synthetic growth medium mimicking cystic fibrosis lung sputum", JOURNAL OF MEDICAL MICROBIOLOGY, vol. 59, no. 9, 3 June 2010 (2010-06-03), pages 1089-1100, XP55619934, ISSN: 0022-2615, DOI: 10.1099/jmm.0.019984-0
- H Yamada ET AL: "Preparation of mycobacteria-containing artificial sputum for TB panel testing and microscopy of sputum smears", The International Journal of Tuberculosis and Lung Disease, 1 August 2006 (2006-08-01), pages 899-905, XP055620471, France Retrieved from the Internet: URL:https://www.ingentaconnect.com/content /iuatld/ijtld/2006/00000010/00000008/art00 013%3bjsessionid=2fsoxldht94mf.x-ic-live-0 2 [retrieved on 2019-09-09]

## Description

The present invention relates to artificial sputum, a method of producing artificial sputum and use of artificial sputum for quality assessment or for training purposes.

Tuberculosis (TB) remains the killer number one among infectious diseases caused by single pathogens. According to the WHO health report 2018, tuberculosis caused estimated 1.3 million deaths among HIV-negative people and 300,000 deaths among HIV-positive people in 2017. WHO estimated that 10.0 million people developed TB. Furthermore, increasing drug resistance of the pathogen *Mycobacterium tuberculosis* complex (MTBC) is a rising problem. In 2017, 639,000 people developed rifampicin (the most effective first-line anti-TB drug) resistant tuberculosis, and of those, 82% had multidrug-resistant tuberculosis (MDR-TB).

Early and reliable diagnosis of TB and its resistance patterns (e.g. MDR-TB) improves treatment outcomes of the patients and allows for their rapid separation from the community for the purpose to interrupt transmission and further spread of the pathogen. Since the vast majority of TB patients suffer from pulmonary tuberculosis, the diagnosis mostly relies on laboratory detection of MTBC in respiratory specimens by means of microscopy after specific staining, of culture after decontamination and enrichment, and of molecular-biological nucleic acid amplification tests (NAT). Respiratory secretions, first and foremost the most frequently encountered human sputum, are biological samples with highly complex and heterogeneous structures very widely differing in macroscopic appearance. Sputum composition is influenced by genetic and environmental factors, type and extent of disease, food and fluid intake, mouth hygiene, and the collection procedure. The composition of the sample matrix has a strong impact on the performance of a diagnostic assay and its validity for MTBC detection. Inhibitors can impact NATs, confounders can influence microscopy, and contaminants can cause invalid culture results.

To support early treatment initiation, laboratory diagnostic needs to be highly sensitive, specific and fast. Efforts are made to establish laboratories with appropriate infrastructure for efficient diagnostics particularly in those countries where the burden of tuberculosis is high. However, constructing and equipping those laboratories is only one aspect while building human resource capacities, assuring and monitoring the quality of diagnostics are at least as important. ISO 15189, the standard of quality management in clinical laboratories, and the European Medical Product law demand for validation, verification, regular internal and external quality controls, i.e. comprehensive quality assurance of all existing and newly established diagnostic tests and procedures.

So far those quality assurance procedures are neither sufficiently standardized nor adequately adjusted to diagnostic needs limiting the comparability of results from study to study and even from laboratory to laboratory. Validation of the sensitivity of a diagnostic assay usually includes the determination of the so-called limit of detection (LoD) of an assay. For this, serial dilutions of MTBC bacteria in a test matrix are applied to the assay of interest to determine the minimal number of bacteria the respective test is able to detect. The manufacturers are free to decide in which test matrix they measure the LoD. They can choose from watery suspensions, separate or pooled human sputum samples, artificial sputum or many other options. As indicated above, the sample matrix can have strong impact on the test outcome making it impossible to retrospectively compare LoDs from different studies.

Although only few basic conditions are defined for the verification of CE marked tests in diagnostic laboratories, the verification procedure certainly should include the reproduction of the LoD in test laboratory. But when trying to do so, the laboratory is promptly confronted with the challenge to use the same or at least a very similar test matrix as the manufacturer which might not be easily available, particularly not when the manufacturer had chosen a biological matrix such as human or pooled sputum.

Internal and external quality controls shall reconstruct diagnostic test conditions as closely as possible in a standardized and reproducible way. In the lack of standardized test matrices diagnostic laboratories frequently use human sputum samples with pre-designed test results for internal controls. Neither the composition of the control matrix nor the concentration of bacteria is known under such conditions.

Test panels of external ring trials are also used for quality control purposes. External quality controls for diagnostic laboratories encompasses shipping of panels of (i) pre-confectioned smears from polyacrylamide or methylcellulose-based artificial sputum for direct staining and microscopy, (ii) suspensions of mycobacteria in methylcellulose for culture inoculation with or without prior decontamination, (iii) suspensions of inactivated bacteria for species identification, and (iv) suspensions of bacteria for susceptibility testing. However, polyacrylamide or methylcellulose-based artificial sputum as previously described by Yamada et al. (Journal of Clinical Microbiology, Oct. 2011, p. 3604-3609) and Rogers and Choi (Journal of Microbial & Biochemical Technology, 2012) are different from natural sputum not only in terms of composition but also in terms of how microbiological organisms, e.g. mycobacteria, behave and survive, which biases results of quality control or validation of diagnostics.

In real diagnostics, doctors send respiratory secretions to laboratories which decontaminate and concentrate samples and subsequently perform microscopy smear, inoculate cultures and extract DNA for nucleic acid amplification tests.

A standardized artificial sputum samples with characteristics very similar to human sputum but easily and always identically reproducible would allow to perform all quality control procedures (i.e. validation, verification, internal and external quality controls) following routine procedures thereby yielding much more meaningful and informative results.

Therefore, there is a strong need of an artificial sputum having a high similarity to natural human sputum and of a standardized production procedure allowing to obtain such sputum in always the same composition and quality in a repeatable manner.

According to the present invention this problem is solved by the method for preparing artificial sputum according to claim 1 and by the artificial sputum according to claim 12.

It is noted that the term "artificial sputum" as used herein excludes all natural sputa.

According to the present invention artificial sputum is prepared by a method comprising the following steps: Step (A) preparing an aqueous solution or dispersion of nutrients and step (B) adding non-human mucin to the aqueous solution or dispersion obtained in step (A) such that the concentration of mucin is in the range of 6 to 18 percent by weight (based on the total weight of the artificial sputum) and the viscosity of the artificial sputum is in a range of 30 to 480 mPa·s.

By preparing first a solution or dispersion of nutrients which are preferably selected to supplement growth of bacteria, before mucin is added, it is easier to obtain a homogenous mixture. Moreover, by adding non-human mucin such that the concentration of mucin is in the range of 6 to 18 percent by weight, based on the total weight of the artificial sputum, the viscosity of the artificial sputum can easily be adapted to that of human sputum, i.e. to a viscosity in the range of 30 to 480 mPa·s. Artificial sputum obtained by this method cannot be distinguished from human sputum by macroscopic analysis, i.e. by the appearance such as color, flow behavior and tendency to form mucus threads. Due to the highly similar appearance compared to human sputum it is very well suited for training in diagnosis of bacteria or quality assessment for any kind of process applying human sputum. It is noted that all viscosity values mentioned herein are measured using a DV2TL Brookfield viscometer equipped with a cone spindle CP40 at 22.5°C with a rotation speed of the spindle being set to 0.5 rpm.

It is preferred that in the method according to the present invention a pasteurization step (C) with the aim to disinfect or sterilize the solution obtained in step (B) is performed. Some applications may not need such sterilizing. However, it is advantageous, since thereby a stock of artificial sputum can be reliably produced and reproduction becomes easier, since the residual bacterial and fungous flora is drastically reduced or even a complete sterilization is achieved.

When it is referred herein to a pasteurization step, this encompasses measures suitable for inactivating bacteria and other pathogens or even completely sterilizing a solution. The pasteurization may be performed by different means.

It is further preferred in the method according to the present invention that the pasteurization step (C) is performed by maintaining the solution at a temperature of 70 °C or higher for a time of 60 seconds or longer. More preferably, the temperature at which the solution is maintained is 75 °C or higher and the time is 5 minutes or longer. The time is more preferably 10 minutes or longer, even more preferably 20 minutes or longer and most preferably 40 minutes or longer. It is also preferred that after addition of mucin in step (B) the temperature is kept below 90°C. In this context it is clear that it is particularly preferred that the sterilizing step (C) is performed by maintaining the solution at a temperature in the range of 70 to 90°C, even more preferred in a range of 75 to 85°C. In step (C) the temperature in the range of 70 to 90°C, more preferably in the range of 75 to 85°C is preferably maintained for a time of 5 minutes or longer. By heating to a temperature of 70 °C or higher for a time of 60 seconds or longer it is possible to significantly reduce the residual bacterial count. By keeping the temperature below 90°C the viscosity of the artificial sputum is not significantly reduced. Using a temperature in the range of 75 to 85°C for a time of 5 minutes or longer, more preferably 10 minutes or longer, even more preferably 20 minutes or longer and most preferably 40 minutes or longer can provide a pronounced reduction of the residual bacterial count while the viscosity of the artificial sputum remains very similar to the viscosity of human sputum.

Another preferred method for performing step (C) is carrying out a sterile filtration. This can reduce the residual bacterial count. However, with the mucin concentration being in the range of 6 to 18 percent by weight, based on the total weight of the artificial sputum, the viscosity of the artificial sputum is quite high and a sterile filtration is cumbersome or even impossible since the filter pores might be clogged. Therefore, pasteurization in step (C) is more preferably carried out by heating as described above.

For obtaining a low residual bacterial count, it is further preferable that in the method according to the present invention the aqueous solution prepared in step (A) is sterilized by autoclave treatment at 105 to 140 °C for a time of 1 minutes to 24 hours at a pressure of 1,1 to 5 bar before step (B) is performed. More preferably the time of the autoclave treatment is in a range of 5 minutes to 12 hours, even more preferably in a range of 10 minutes to 5 hours. By autoclaving the solution prepared in step (A) all unwanted microbiological contaminations can be neutralized so that after step (B) is performed less harsh sterilizing conditions can be applied or sterilizing can be even completely omitted after step (B) to produce a sterile artificial sputum. In this regard it is noted that it is particularly preferred that the autoclave treatment after step (A) is performed and that step (C) is performed after step (B) as described above.

For closely mimicking the macroscopic and microscopic appearance of human sputum it is particularly preferred that in the method according to the present invention the amount of mucin added in step (B) is such that the final concentration of mucin is in the range of 8 to 16 percent by weight, even more preferably in the range of 10 to 14 percent by weight, based on the total weight of the artificial sputum

Mucin from porcine or mucin from bovine is preferably used in the method according to the present invention. These mucins are easily available and are suitable to provide an artificial sputum very similar in appearance to human sputum. From a stand point of availability and price it is more preferable to use mucin from porcine, in particular mucin from porcine stomach type II.

It may be preferable that in the method according to the present invention DNA is added, wherein the DNA is from another species than the source of origin of the non-human mucin. Addition of DNA further increases the similarity of the artificial sputum according to the present invention compared to natural human sputum. Even though the addition of DNA increases the similarity in regard to macroscopic and microscopic appearance, a differentiation possibility on a molecular level is provided, since the DNA and the non-human sputum are from different species.

In the method according to the present invention it is preferable to add at least one catalase in step (A) and/or step (B). By adding a catalase oxidative stress in the artificial sputum is reduced which is favorable for survivability of mycobacteria, such as *Mycobacterium tuberculosis.*

It is further preferred that that in the method according to the present invention a growth supplement for bacteria is added. Most preferably a growth supplement for mycobacteria is added.

For improving homogenization of nutrients in the artificial sputum it is preferable that in the method according to the present invention glycerin is added. By adding glycerin crystal formation is reduced or completely avoided when the artificial sputum is frozen. This is advantageous when artificial sputum is spiked with bacteria and frozen samples thereof are send to laboratories, e.g. for round robin tests. In this regard glycerin is preferably added in an amount so that the final concentration of glycerin is in the range of 0.5 to 4 ml per 100 ml of artificial sputum, more preferably in the range of 1 to 3 ml per 100 ml of artificial sputum.

Preferably the pH value is adjusted to a value in the range of from 5.0 to 8.0, preferably in the range of from 5.5 to 7.5, most preferably in the range of 6 to 7.0, before step (B) is performed. With a pH value in the specified range mycobacteria have a good survivability.

The present invention also relates to artificial sputum obtainable by the method described in the claims and above.

Preferably the artificial sputum according to the present invention has a viscosity in the range of from 75 to 300 mPa·s, even more preferably in the range of from 100 to 275 mPa·s. With a viscosity in the said range particularly the macroscopic appearance is very similar to human sputum so that the artificial sputum having such a viscosity is very well suited for training purposes.

It is further preferable that the artificial sputum according to the present invention contains a suspension of mononuclear cells, such as human mononuclear cells, and background bacteria, i.e. it is preferable that such a suspension is added in the method for preparing artificial sputum described in the claims and above.

In addition, the present invention concerns the use of artificial sputum according to the present invention in quality assessment, for example validation, verification and quality control of analytic assays on sputum, or for training in bacteriological diagnostics. By using the artificial sputum according to the invention it is possible to provide realistic conditions for quality assessment and training which otherwise could only be provided using human sputum.

It is preferable to use of artificial sputum according to the present invention for training in bacteriological diagnostics, wherein the bacteria are from a family selected from the group consisting of Mycobacteriaceae.

The artificial sputum according to the present invention turns out to be particularly useful for performing external quality assessment, since the appearance is so close to human sputum that the person performing the measurements during external quality assessment is under impression of analyzing a real sample of human sputum.

Due to a good survivability of *Mycobacterium tuberculosis* it is particularly preferred to use the artificial sputum according to the present invention for an external quality assessment concerning TB diagnostics by detection of *Mycobacterium tuberculosis* complex. The external quality assessment is preferably performed in the context of ring trials.

Also the use of artificial sputum according to the present invention for validating and evaluating diagnostic tests or devices is preferred, since it can reliably be reproduced and easily be provided in quantities that allow performing all experiments required to obtain sufficient data to examine the test or device with adequate statistical power.

Hereafter, the invention will be described in more detail also by referring to Figures and Examples which, however, do not limit the present application.
- Fig. 1: shows viscosity of artificial sputum according to the present invention with different mucin concentration.
- Fig. 2: shows photographs of blood agar plates after incubation at 37 °C for 48 hours, to which artificial sputum samples according to the present invention were inoculated which were a) not sterilized; b) pasteurized at 70°C for 45 minutes; c) pasteurized at 80°C for 45 minutes; and d) pasteurized at 90°C for 45 minutes.
- Fig. 3: shows photographs of artificial sputum samples according to the present invention dropping from a pipet tip, wherein the artificial sputum samples were a) not sterilized; b) sterilized at 70°C for 45 minutes; c) sterilized at 80°C for 45 minutes; and d) sterilized at 90°C for 45 minutes.
- Fig. 4: shows micrographs taken from a) human sputum and b) artificial sputum according to the present invention, wherein a Ziehl-Neelsen staining has been performed.
- Fig. 5: shows micrographs taken from a) human sputum, b) artificial sputum according to the present invention, c) artificial sputum based on methyl cellulose, d) artificial sputum based on polyacrylamide, wherein a Ziehl-Neelsen staining has been performed.
- Fig. 6: shows micrographs taken from a) human sputum, b) artificial sputum according to the invention, c) artificial sputum based on methylcellulose, d) artificial sputum based on polyacrylamide, wherein a Gram staining has been performed.
- Fig. 7: shows survivability of *Mycobacterium tuberculosis* in artificial sputum according to the present invention at different temperatures.
- Fig. 8: shows the analytical sensitivity of the PCR-based test GeneXpert MTB/RIF Ultra (Cepheid) in human sputum (HS), artificial sputum according to the present invention (MUCAS) and physiological saline solution (Saline). Sensitivity is expressed as colony forming units per ml (CFU/ml).
- Fig. 9: shows the analytical sensitivity of FluoroType-MTB (Hain Lifescience) in human sputum (HS), artificial sputum according to the present invention (MUCAS) and physiological saline solution (Saline). Sensitivity is expressed as colony forming units per ml (CFU/ml).

In the subsequent examples the following materials were used:
- DNA from salmon sperm (obtainable from Sigma-Aldrich, product number 31149,)
- Mucin from porcine stomach Type II (obtainable from Sigma-Aldrich, product number M2378)
- NaOH (obtainable from Sigma-Aldrich)
- Middlebrook 7H9 Mycobacteria Base ADC Enrichment Medium (Powder)
- Bidest. glycerol (obtainable from VWR)
- OADC (Oleic Albumin Dextrose Catalase) enrichment (BD)
- Casitone (BD Bacto)
- Acrylamide (Roth)
- N,N'-methylenebisacrylamide (Roth)
- TBE (Tris/Borate/EDTA) buffer (Roth)
- 10% Ammonium peroxodisulfate (Roth, 2.5 g dissolved in 25 ml distilled H₂O)
- Tetramethylethylenediamine (TEMED obtainable from Roth)
- Methylcellulose

The bacterial strains summarized in the below table 1 were used to prepare background flora in artificial sputum samples.

**Table 1:**

| **DSMZ number** | **Lot number** | **Strain designation** |
|---|---|---|
| 4691 | 0213 | *Neisseria lactamica* |
| 26920 | 0213 | *Streptococcus rubneri* |
| 19150 | 0407 | *Moraxella lincolnii* |

Samples of the strains listed in table 1 are deposited at the Leibniz-Institut DSMZ - Deutsche Sammlung von Mikroorganismen und Zellkulturen GmbH (DSMZ) under the DSMZ number also indicated in table 1 and are available to purchase from the DSMZ.

### Preparation of artificial sputum:

### Example 1: Preparation of mucin based artificial sputum:

In a 100 ml glass flask with a magnetic stirrer 0.24 g of 7H9 powder and 0.05 g of casitone were mixed with 0.2 g (0.4% w/v) salmon DNA. The mixture was dissolved in 49 mL of distilled water. Thereafter 1 mL glycerin was added and the mixture was stirred until all ingredients were completely dissolved. Thereafter the pH was adjusted to 7.0 using NaOH. The obtained solution was sterilized by autoclaving at 121 °C for 20 min at a pressure of 2 bar.

After autoclaving 6 g mucin (porcine mucin, type II) was added and the resulting mixture was stirred until the mucin was completely dissolved. The resulting mixture was pasteurized at 80 °C for 45 minutes in a preheated water bath.

### Example 2: Preparation of mucin based artificial sputum containing OADC (Oleic Albumin Dextrose Catalase) growth supplement:

In a 100 ml glass flask with a magnetic stirrer 0.24 g of 7H9 powder and 0.05 g of casitone were mixed with 0.2 g (0.4% w/v) salmon DNA. The mixture was dissolved in 44 mL of distilled water. Thereafter 1 mL glycerin was added and the mixture was stirred until complete solubilisation was achieved. Thereafter the pH was adjusted to 7.0 using NaOH. The obtained solution was sterilized by autoclaving at 120 °C for 20 min at a pressure of 2 bar.

After autoclaving 6 g (12 % w/v) mucin were added and the resulting mixture was stirred until complete solution was achieved. The resulting mixture was sterilized at 80 °C for 45 minutes in a preheated water bath. After cooling down to room temperature 5 ml of an OADC growth supplement was added.

### Example 3: Inoculation of mucin based artificial sputum with mononuclear cells and background flora:

A suspension of human mononuclear cells and background bacteria was obtained by the following procedure: 1 ml aliquots of each of *S. rubnerii* (approximately McF 5), *N. lactamica* (approximately McF 5) and *M lincolnii* (approximately McF 5) were thawed. Thereafter the bacteria were centrifuged at 10,000 rpm for 2 min and the resulting supernatant was discarded to obtain pellets containing the bacteria. A 1 ml aliquot containing human mononuclear cells (180·10⁶ cells/ml) was thawed and centrifuged at 3,000 rpm for 10 min and the resulting supernatant was discarded to obtain a pellet containing human mononuclear cells. The pellet containing human mononuclear cells was resuspended in 1 ml of 0.85% saline. After resuspension of human mononuclear cells was complete, the pellets containing *S. rubnerii* (McF 5), *N. lactamica* (McF 5) and *M lincolnii* (McF 5) were subsequently resuspended one after another.

1 ml of the suspension containing background bacteria *S. rubnerii* (McF 5), *N. lactamica* (McF 5) and *M lincolnii* (McF 5) and human mononuclear cells was added to 9 ml of the above obtained mucin based artificial sputum according to Example 2. Sterile glass beads (6 mm in diameter) were added and the mixture was vortexed for 2 minutes.

### Example 4: Preparation of methylcellulose based artificial sputum:

The content of one hen egg was added to a 50 ml plastic tube and homogenized by vigorously shaking the tube to emulsify the egg. 1/20 volume of the emulsified egg was transferred into a fresh 50 ml plastic tube containing 30 ml of 1% methylcellulose solution. The volume was finally adjusted to 50 ml using 1% methylcellulose solution and mixed well by inverting several times. To obtain a 1% methylcellulose solution, 0.5 g of methylcellulose were previously dissolved in 50 ml sterile and distilled water.

Background flora and mononuclear cells were prepared and spiked into methylcellulose based artificial sputum according to Example 3.

### Example 5: Preparation of polyacrylamide based artificial sputum:

11.1 g of acrylamide and 0.3 g of N,N'-methylenebisacrylamide were dissolved in 50 ml distilled water to prepare a stock solution.

0.875 ml of the above stock solution was diluted with 8.125 ml of distilled water and 1 ml of TBE buffer and 50 µl of 10% ammonium peroxodisulfate solution were added and the components were thoroughly mixed before 4 µl TEMED were added and the mixture was allowed to polymerize over a period of 1 to 2 days.

Background flora and mononuclear cells were prepared and spiked into polyacrylamide based artificial sputum according to Example 3. In contrast to Example 3 and 4, Background flora, MTB and mononuclear cells were spiked one day prior to experiment, since polymerization of low percent polyacrylamide was allowed to occur over a period of 24h at room temperature.

### Macroscopic comparison of sputa described above:

The above described artificial sputa were evaluated with regard to viscous behavior, color, and foam formation. These evaluations were made by visual inspection of samples of artificial sputa of Examples 1 to 5 placed in transparent plastic tubes. It was examined, how the samples of artificial sputa of Examples 1 to 5 form mucus threads when the artificial sputa were dripping from a pipette.

The mucin based artificial sputa of Examples 1 to 3 did not differ significantly from each other in regard to viscous behavior, color and foam formation. Sputum according to Example 3 had a slightly lower viscosity due to the reduced mucin content in comparison to sputa of Examples 1 and 2. No excessive foam formation could be observed and the color was brown-yellowish, comparable to human sputum.

The methylcellulose based sputum according to Example 4 has a lighter yellowish color and a strong tendency to foam formation was observed. When dripping from a pipette, single drops fall without forming visible threads.

The polyacrylamide based artificial sputum of Example 5 was transparent. Foam formation was comparable to the sputa of Examples 1 to 3 and human sputum. The viscosity was significantly increased compared to Examples 1-4. This artificial sputum formed a polyacrylamide clump and mucus threads in a much stronger manner than the sputa of Examples 1 to 3 and human sputum.

The macroscopic comparison shows that the mucin based artificial sputa of Examples 1 to 3 has an appearance identical to human sputum. This is not the case for other types of artificial sputum, such as methylcellulose based artificial sputum of Example 4 or polyacrylamide based sputum of Example 5.

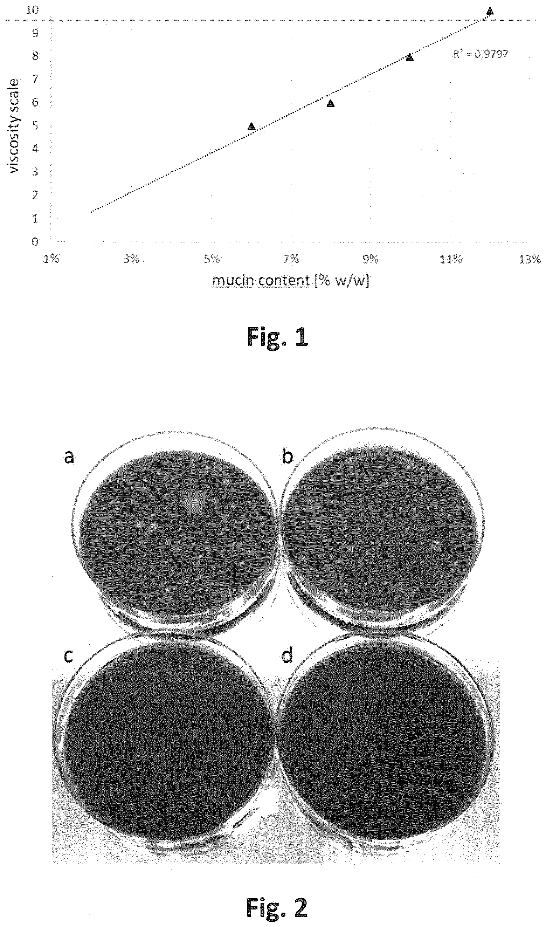

The influence of mucin concentration in artificial sputum on the viscous behavior was examined by preparing artificial sputa according to Example 1 but the mucin concentration was changed to 6, 8, 10 and 12 g/100 g sputum. 2 ml of the so prepared artificial sputum samples were placed in 15 ml transparent plastic vials and it was compared how the samples flow inside the plastic vials and adhere to the inner walls of the plastic vials when the vials were inverted. In addition, the sputum samples were taken up with a 1 ml disposable Pasteur pipette and it was examined how mucin threads are formed and how much volume remained in the tip of the pipette. Also a comparison with human sputum was made. In Fig. 1 a comparison of the viscous behavior of artificial sputa with differing mucin content is provided. The viscosity scale is an arbitrary scale in which human sputum is set to 9.5, as indicated by the dashed horizontal line.

Further viscosity studies were made using artificial sputa having the composition as indicated in table 2. The artificial sputa were prepared as described above for Example 1. The artificial sputa of Examples 1-2, 2-2 and 3-2 did not contain DNA. The artificial sputa of Examples 1-3, 2-3 and 3-3 did not contain glycerol. For comparison, an artificial sputum medium (ASM) was prepared as described by Sriramulu, 2010 Nature protocol exchange (doi:10.1038/protex.2010.212):
80 ml of distilled water were added to a 250 ml glass flask including a magnetic stirrer and the flask was placed on a magnetic stirrer plate. Under constant stirring, mucin, DNA, salts (NaCl and KCI) and Tris base were added one after another and stirring was continued until all ingredients were dissolved. The pH was adjusted to 7 using Tris base. Thereafter water was added until the total volume was 100 ml. The obtained mixture was autoclaved at 121 °C for 15 min. After cooling down, 0.5 ml of egg yolk were added to the mixture. The mixture was stored in a refrigerator at 1 to 10°C.

Viscosity measurements were performed using a DV2TL Brookfield viscometer equipped with a cone spindle CP40 at 22.5°C. Rotation speed of the spindle was set to 0.5 rpm. For data collection, single point averaging was used with end condition time being 30 seconds and single point averaging duration was 20 seconds. The single data point collected is an average of the measured data from 10 seconds to 30 seconds. Per measurement 500 µl of artificial sputum were used. The observed viscosities are summarized in the below table 2.

In the below table 2 all contents are in wt.-% based on the total weight of the artificial sputum, missing percent to 100 wt.-% are water. The unit for the viscosity is in mPas·s.

**Table 2:**

| Content | Ex. 1- | Ex. 1- | Ex. 1- | Ex. 2- | Ex. 2- | Ex. 2- | Ex. 3- | Ex. 3- | Ex. 3- | ASM |
|---|---|---|---|---|---|---|---|---|---|---|
| | 1 | 2 | 3 | 1 | 2 | 3 | 1 | 2 | 3 | |
| Mucin | 6% | 6% | 6% | 12% | 12% | 12% | 18% | 18% | 18% | 0.5% |
| DNA | 0.4% | - | 0.4% | 0.4% | - | 0.4% | 0.4% | - | 0.4% | 0.4% |
| Glycerol | 2% | 2% | - | 2% | 2% | - | 2% | 2% | - | - |
| Casitone | 0.1% | 0.1% | 0.1% | 0.1% | 0.1% | 0.1% | 0.1% | 0.1% | 0.1% | - |
| 7H9 | 0.5% | 0.5% | 0.5% | 0.5% | 0.5% | 0.5% | 0.5% | 0.5% | 0.5% | - |
| DTPA | - | - | - | - | - | - | - | - | - | 0.59% |
| NaCl | - | - | - | - | - | - | - | - | - | 0.5% |
| KCI | - | - | - | - | - | - | - | - | - | 0.22% |
| Tris | - | - | - | - | - | - | - | - | - | 0.18% |
| Egg yolk | - | - | - | - | - | - | - | - | - | 0.5% |
| Asn | - | - | - | - | - | - | - | - | - | 0.03% |
| η | 38.0 | 42.3 | 31.3 | 158.2 | 222.0 | 259.0 | 462.9 | 348.9 | 340.9 | 4.3 |

Based on the observed flowing behavior, thread formation and the viscosity measurements, it was found that a mucin concentration around 12 g mucin per 100 g sputum (i.e. the artificial sputum according to Example 1) had virtually identical viscous behavior as human sputum. Over a concentration range of 6 g to 18 g mucin per 100 g mucin, human sputum was imitated very well.

In contrast, mucin concentrations below 8 g/100 g sputum have a lower viscosity compared to human sputum. It can be expected that for concentrations above 18 g/100 g sputum the viscosity increases to such an extent that difficulties in pipetting can occur. A very good similarity to human sputum is obtained with a mucin concentration in the range of 8 to 18 g/100 g sputum, particularly with a mucin concentration in the range of 10 to 16 g/100 g sputum.

### Pasteurization / Sterilization:

How pasteurization conditions influence bacterial count and viscous behavior of mucin based artificial sputum was examined by preparing artificial sputum as described in Examples 1 and 2. However, in the step of pasteurization for 45 minutes in a water bath temperature was 70 °C, 80 °C or 90 °C or no pasteurization at all was performed after addition of mucin.

The obtained mucin samples were plated on blood agar to determine the residual bacterial flora. Photographs of the corresponding blood agar plates after 48 hours at 37 °C are provided in Fig. 2a to 2d. Without pasteurization it was observed that a significant amount of colonies grew on the blood agar substrate (see Fig. 2a). In case the pasteurization was performed at 70 °C for 45 minutes, significantly fewer colonies were observed (see Fig. 2b). When the temperature during pasteurization treatment was increased up to 80 °C, no colonies at all were observed, indicating that all bacterial flora was successfully inactivated at 80 °C (see Fig. 2c). As can be expected from the results of the pasteurization at 80 °C, the pasteurization at 90 °C was also successful (see Fig. 2d).

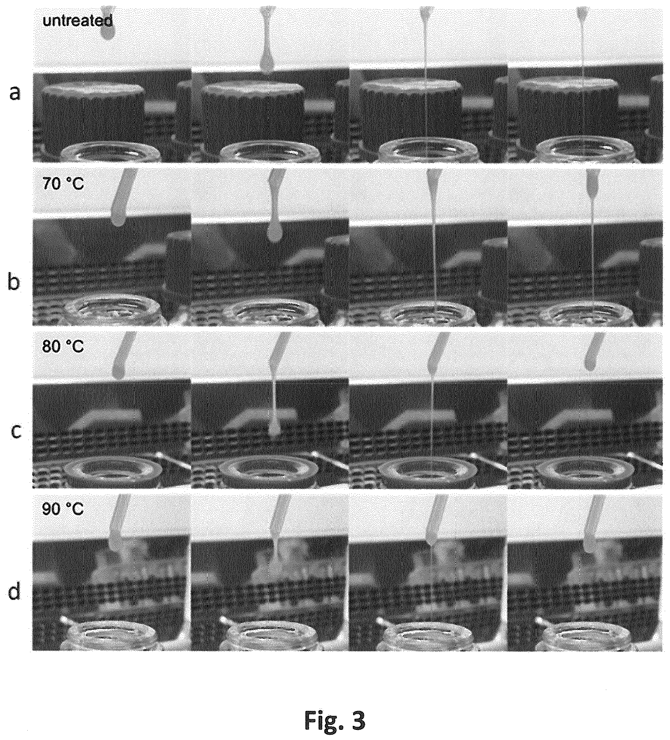

It was further examined how the viscous behavior of the artificial sputum was influenced by the temperature of the pasteurization treatment by lifting some of the artificial sputum with a 1 ml disposable Pasteur pipette and letting it drop from the tip of the pipette. Photographs from how drops of the artificial sputum dropped from the tip are provided in Fig. 3a to 3d. Fig. 3a shows how the artificial sputum that has not been pasteurized dropped from the pipette. Photographs of artificial sputum being partially pasteurized at 70°C dropping from the tip are shown in Fig. 3b. Corresponding photographs of artificial sputum dropping from a pipette tip after pasteurization at 80°C and 90°C, respectively, are shown in Fig. 3c and 3c. It was found that when the pasteurization temperature was 70°C, the artificial sputum dropped almost identical to when no pasteurization treatment was performed (see Fig. 3a and 3b). When increasing the temperature to 80°C, the viscosity was slightly reduced but still comparable to human sputum (see Fig. 3c). When the temperature of pasteurization treatment was further increased to 90°C, a significant reduction of the sputum viscosity was observed (see Fig. 3d). Therefore, if pasteurized artificial sputum is required to have viscous behavior similar to human sputum it is highly preferable to perform sterilization treatment at a temperature in the range of 75°C to 85°C.

### Microscopic comparison of sputa described above:

Artificial sputa of Examples 4 and 5 were inoculated with background bacteria and human mononuclear cells as in Example 3. The inoculated sputa of Examples 3 to 5 and human sputum were spiked with the *Mycobacterium tuberculosis* (MTB) strains H37Ra (ATCC 25177) and H37Rv (ATCC 27294) so that each sample had an identical concentration of colony forming units of MTB bacteria. MTB strains can be purchased at the American type culture collection (ATCC).

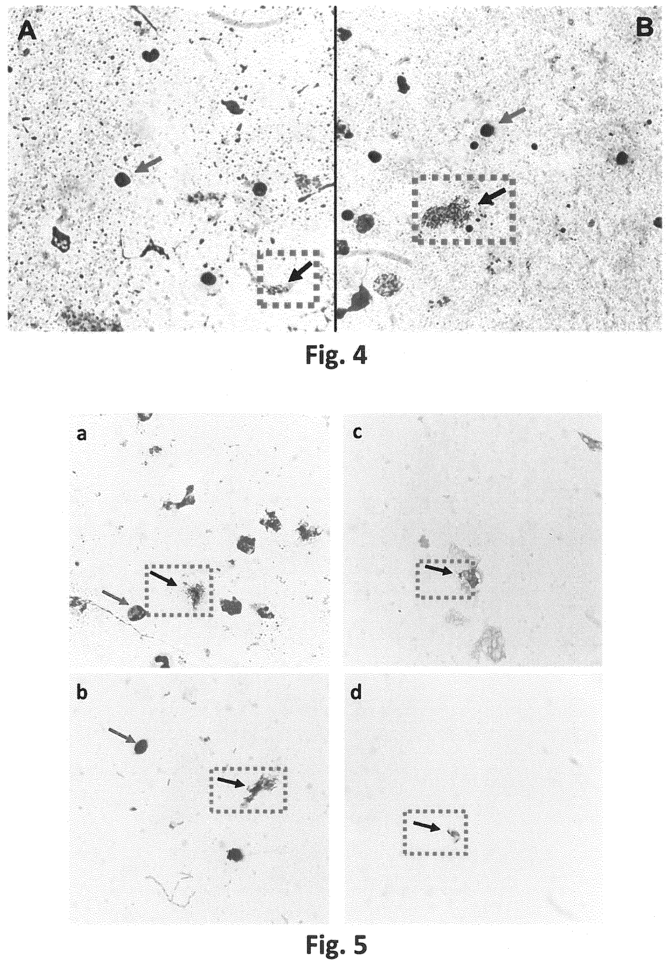

The artificial sputa inoculated with background bacteria and human mononuclear cells and being spiked with MTB bacteria were subjected to Ziehl-Neelsen staining and Gram staining. Micrographs of stained samples of artificial sputum are shown in Fig. 4 to 6. Fig. 4a and 4b show a direct microscopic comparison of human sputum and artificial sputum according to Example 3 (inoculation was only performed with mononuclear cells and not with the bacterial background) after being spiked with MTB (H37Ra). The dashed box and the black arrows indicate mycobacteria and the grey arrows indicate mononuclear cells. It is noted that not all visible mononuclear cells in Fig. 4a and 4b are indicated by a grey arrow. By microscopic analysis it is not possible to distinguish the human sputum from the artificial sputum according to Example 3.

A comparison of all four examined samples, i.e. inoculated sputa of Examples 3 to 5 and human sputum spiked with MTB (H37Rv) and subjected to Ziehl-Neelsen staining is provided by micrographs shown in Fig. 5a to 5d. Fig. 5a shows a micrograph obtained from the sample with human sputum. Fig. 5b shows a micrograph obtained from the sample with mucin based artificial sputum according to the present invention. Fig. 5c shows a micrograph obtained from methylcellulose based artificial sputum. Fig. 5d shows a micrograph obtained from polyacrylamide based artificial sputum. With the artificial sputum according to the present invention it is possible to perform Ziehl-Neelsen staining identical to human sputum (see Fig 5a and 5b). With the Ziehl-Neelsen stained methylcellulose based sputum of Example 4 staining (see Fig 5c), the quality of the smear was significantly worse compared to human sputum or to the sputum according to Example 3 since no mononuclear cells were observed. In the Ziehl-Neelsen stained polyacrylamide based sputum of Example 5 (see Fig 5d) also no mononuclear cells could be identified. A likely explanation of this major difference of methycellulose and polyacrylamide based sputa to human or the artificial sputum of example 3 is that the integrity of mononuclear cells disrupts in the environment methycellulose or polyacrylamide solutions.

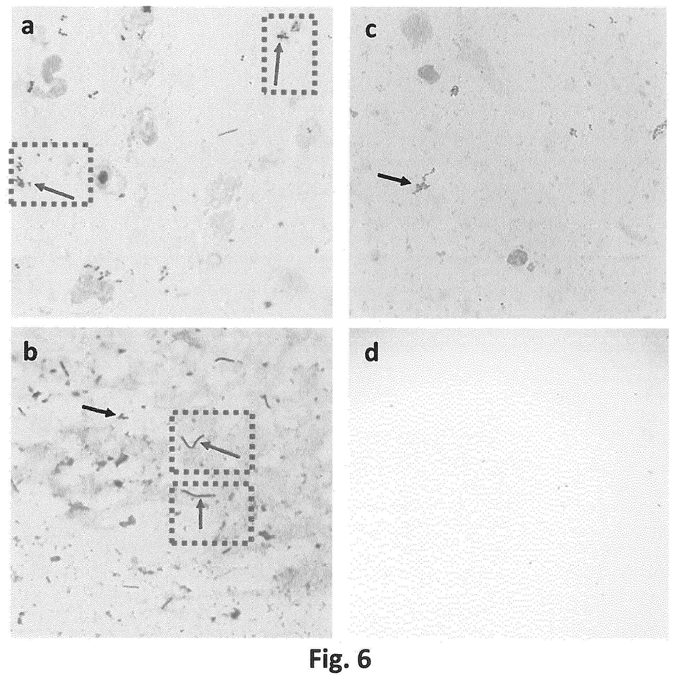

By using Gram-staining, it is possible to distinguish bacteria regarding their form and integrity as well as their cell wall architecture. While Gram positive coccus (for example Staphylococci) and rod bacteria (for example Corynebacteria) appear dark bluish, roundish and rod-shaped, respectively, Gram negative cocci and rods appear with a clear red color. Head-to-head comparing the Gram-stain of all four types of sputa (artificial sputa according to Examples 3 to 5 and human sputum, all spiked with the same suspensions of Gram-positive, Gram-negative as well as MTB-H37Rv bacteria in identical concentrations) also revealed major differences of methycellulose and acrylamide based artificial to human or the artificial sputum according to the present invention (see Fig. 6a-d). Fig. 6a, 6b, 6c, and 6d show micrographs obtained from human sputum, mucin based artificial sputum according to the present invention, methylcellulose, and polyacrylamide based artificial sputum, respectively. Gram positive bacteria are marked with grey arrows and a dashed box, Gram negative bacteria with a black arrow only. With the artificial sputum according to the present invention, the overall appearance is indistinguishable from human sputum and Gram positive and Gram negative bacteria can be easily identified (see Fig 6a and 6b). In the methylcellulose based sputum of Example 4 all bacteria appear reddish like Gram negative while no Gram positive bacterium became visible (see Fig 6c) and in the polyacrylamide based sputum of Example 5 Gram no bacteria became visible at all (see Fig 6d).

This demonstrated that the artificial sputum according to the present invention yields results identical to human sputum no matter which of the microbiological standard stains is applied. This makes the artificial sputum according to the present invention a very suitable training material for microscopists who shall analyze human sputum after Ziehl-Neelsen or Gram staining.

An overview over the microscopy results is provided in the below table 3. In this table smear quality identical to human sputum is indicated by "+++", lower quality is indicated by "++", even lower quality is indicated by "+" and very low quality for example due to the loss of all bacteria is inidated by "-".

**Table 3**

| Type of Sputum | Ziehl-Neelsen staining | Gram staining |
|---|---|---|
| Human Sputum | +++ | +++ |
| MUCAS | +++ | +++ |
| Methylcellulose | ++ | + |
| Polyacrylamide | + | - |

### Survivability:

For performing external quality assessments (EQA) such as ring trials it is required the test bacteria survive long enough in the test medium to allow the participating test laboratories to recover them after shipment. Usually, shipment of bacteriological EQA test panels lasts up to five days; thus the test bacteria need to survive at least several days in the sample.

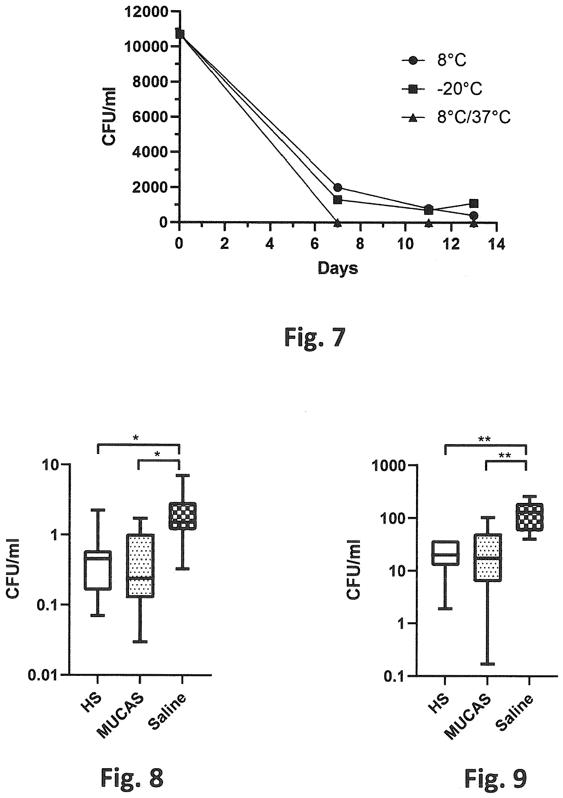

To test survivability of MTB strain H37Ra in the artificial sputum according to the present invention, artificial sputum was prepared as described in Example 3, however inoculation was performed only with mononuclear cells and not with the bacterial background. The spiking with MTB strain H37Ra was adapted so that each sample contained around 11,000 colony forming units per ml (CFU/mL). Samples were kept at constant temperatures of 8°C and -20°C for 13 days. The temperature of a further sample was changed each day from 8°C to 37°C and back, i.e. temperature was kept for one day at 8°C, the next day it was kept for one day at 37°C, the next day it was kept again for one day at 8°C and so on. After day 7, 11 and 13 the number of colony forming units per ml was determined by plating on selective 7H11 agar plates and counting colonies after 3 weeks of incubation at 37°C. The results can be seen from the graph in Fig. 7.

When storage temperature was kept constant at 8°C and -20°C the MTB strain H37Ra survived for at least 13 days. This shows that it is possible to use the artificial sputum according to the present invention for external quality assessment of laboratories, particularly for analysis of MTB.

### Molecular biological analysis:

It was further evaluated, how well the artificial sputum according to the present invention works in quality assessment of microbiological tests as well as of machines used for molecular biological analysis. To do so, for each of the artificial sputum described above two samples were prepared including *Mycobacterium tuberculosis* at a very low concentration close to the estimated limit of detection of the test machine. Thereafter, it was tested whether the GeneXpert MTB/RIF *ultra* (Cepheid, USA) identifies the samples as positive.

When using the artificial sputum according to the present invention it was possible to identify both samples containing *Mycobacterium tuberculosis.* When the methylcellulose based sputum was used, none of the MTB containing samples was identified positive. In the polyacrylamide based sputum only 1 of the 2 MTB containing samples could be identified. This demonstrates that the artificial sputum according to the present invention can be used to evaluate the quality of molecular biological analysis systems and is a better test medium for this purpose than methylcellulose or polyacrylamide based sputa.

It was further tested how sensitive molecular biological diagnostic tests perform in artificial sputum according to the present invention compared to human sputum and physiological saline solution. The analytical sensitivity of GeneXpert MTB/RIF Ultra, i.e. the limit of detection, in human sputum and the artificial sputum according to the present invention was around 1.0 CFU/mL and elevated when saline was used. The limit of detection using GeneXpert MTB/RIF Ultra is displayed in Fig. 8 for human sputum (HS, n=10), artificial sputum according to the present invention (MUCAS, n=9) and saline (Saline, n=6). In Fig. 8 "*" indicates a p-value of p≤0.05 for an One-Way-ANOVA with Tukey's multiple comparisons test. The FluoroType-MTB performed also comparable when human sputum and the artificial sputum according to the present invention were used with a limit of detection of roughly 25 CFU/mL (Fig. 9). When physiological saline was used FluoroType-MTB was significantly less sensitive with a limit of detection of around 130 CFU/mL. This demonstrates that the artificial sputum according to the present invention can be used to validate and conduct molecular biological tests. The artificial sputum according to Example 3 leads to the same test results as tests performed with human sputum and was superior to saline solution. The limit of detection of FluoroType-MTB is displayed in Fig. 9 for human sputum (HS, n=6), artificial sputum according to the present invention (MUCAS, n=9) and saline (Saline, n=9). In Fig. 9 "**" indicates a p-value of p≤0.01 for an One-Way-ANOVA with Tukey's multiple comparisons test.

Mucin based artificial sputum according to Example 3 was spiked with MTB (H37Rv) at different concentrations and decontaminated using the standard N-acetyl-L-Cysteine (NALC)-NaOH method as recommended by WHO for TB diagnostics. The procedure liquefies the sputum with NALC and kills background flora by NaOH so that overgrowth of the MTB culture by rapidly proliferating microorganisms is inhibited. The mycolic acids in the cell wall protect MTB bacteria against destruction from NaOH. The thus decontaminated sputum is then inoculated in MGIT- and LJ tubes and incubated for eight weeks at 37°C. For each concentration of MTB bacteria in the test-sputum six samples were analyzed. The results of MGIT- and LJ cultures are summarized in the below table 4.

**Table 4:**

| H37Rv Concentration CFU/ml | Positive MGIT-cultures | Positive U-cultures |
|---|---|---|
| 10⁴ | 100% | 100% |
| 10³ | 100% | 100% |
| 10² | 100% | 33% |
| 10¹ | 100% | 17% |
| 10⁰ | 33% | 0% |
| 0 | 0% | 0% |

As demonstrated by the results summarized in table 4, when using MGIT it is possible to determine all samples positive using MTB concentrations as low as 10 CFU/ml. Even with only 1 CFU/ml 33% of the samples could be identified positive. For LJ-cultures it was possible to identify 100% of the LJ-cultures positives at concentrations as low as 1,000 CFU/ml. Even for concentrations as low as 10 CFU/ml it was possible to identify positive samples using LJ-cultures.

This demonstrates that the artificial sputum is well suited for external quality assessment of MGIT- and LJ-cultures.

## Claims

1. Method for preparing artificial sputum, comprising the following steps:
Step (A) preparing an aqueous solution or dispersion of nutrients and
Step (B) adding non-human mucin to the aqueous solution or dispersion obtained in step (A) such that the concentration of mucin is in the range of 6 to 18 percent by weight, based on the total weight of the artificial sputum, and the viscosity of the artificial sputum is in a range of 30 to 480 mPa·s.

2. Method according to claim 1, wherein the method further comprises a pasteurization step (C) pasteurizing the solution obtained in step (B).

3. Method according to claim 2, wherein the pasteurization step (C) is performed by maintaining the solution at a temperature of 70 °C or higher for a time of 60 seconds or longer.

4. Method according to any of the preceding claims, wherein the aqueous solution prepared in step (A) is sterilized by autoclaving at 105 to 140 °C for a time of 1 minutes to 24 hours at a pressure of 1,1 to 5 bar before step (B) is performed.

5. Method according to any of the preceding claims, wherein after addition of mucin in step (B) temperature is kept below 90 °C.

6. Method according to any of the preceding claims, wherein the amount of mucin added in step (B) is such that the final concentration of mucin is in the range of 8 to 16 percent by weight, preferably in the range of 10 to 14 percent by weight, based on the total weight of the artificial sputum

7. Method according to any of the preceding claims, wherein the non-human mucin is mucin from porcine or mucin from bovine.

8. Method according to any of the preceding claims, wherein at least one catalase is added in step (A) and/or step (B).

9. Method according to any of the preceding claims, wherein the artificial sputum further comprises a growth supplement for mycobacteria.

10. Method according to any of the preceding claims, wherein the pH value is adjusted to a value in the range of from 5.0 to 8.0, preferably in the range of from 5.5 to 7.5, before step (B) is performed.

11. Method according to any of the preceding claims, wherein the amount of mucin is adjusted such that the artificial sputum has a viscosity is in the range of from 75 to 300 mPa·s, preferably in the range of from 100 to 275 mPa·s.

12. Artificial sputum obtainable by the method according to any of the preceding claims.

13. Use of artificial sputum according to claim 12 in quality assessment or for training in bacteriology.

14. Use of artificial sputum according to claim 13, wherein the bacteria are from a family selected from the group consisting of Mycobacteriaceae.

15. Use of artificial sputum according to claim 13 or 14, wherein the quality assessment is external quality assessment, preferably an external quality assessment of TB diagnostics by detection of *Mycobacterium tuberculosis* complex, preferably in ring trials and/or quality assessment for evaluating and testing diagnostic tests or devices.

## Patentansprüche

1. Verfahren zur Herstellung von künstlichem Sputum, umfassend die folgenden Schritte:
Schritt (A): Herstellen einer wässrigen Lösung oder Dispersion von Nährstoffen und
Schritt (B): Hinzufügen von nicht-menschlichem Mucin zu der in Schritt (A) erhaltenen wässrigen Lösung oder Dispersion, so dass die Konzentration an Mucin im Bereich von 6 bis 18 Gewichtsprozent liegt, bezogen auf das Gesamtgewicht des künstlichen Sputums, und die Viskosität des künstlichen Sputums in einem Bereich von 30 bis 480 mPa·s liegt.

2. Verfahren nach Anspruch 1, wobei das Verfahren zudem einen Pasteurisierungsschritt (C): Pasteurisieren der in Schritt (B) erhaltenen Lösung umfasst.

3. Verfahren nach Anspruch 2, wobei der Pasteurisierungsschritt (C) durchgeführt wird, indem die Lösung für einen Zeitraum von 60 Sekunden oder länger bei einer Temperatur von 70 °C oder höher gehalten wird.

4. Verfahren nach einem der vorangehenden Ansprüche, wobei die in Schritt (A) hergestellte wässrige Lösung durch Autoklavieren bei 105 bis 140 °C für einen Zeitraum von 1 Minute bis 24 Stunden bei einem Druck von 1,1 bis 5 bar sterilisiert wird, bevor Schritt (B) durchgeführt wird.

5. Verfahren nach einem der vorangehenden Ansprüche, wobei die Temperatur nach dem Hinzufügen des Mucins in Schritt (B) unter 90 °C gehalten wird.

6. Verfahren nach einem der vorangehenden Ansprüche, wobei die Menge an in Schritt (B) zugegebenem Mucin derart ist, dass die Endkonzentration an Mucin im Bereich von 8 bis 16 Gewichtsprozent liegt, bevorzugt im Bereich von 10 bis 14 Gewichtsprozent, bezogen auf das Gesamtgewicht des künstlichen Sputums.

7. Verfahren nach einem der vorangehenden Ansprüche, wobei das nichtmenschliche Mucin Schweine-Mucin oder Rinder-Mucin ist.

8. Verfahren nach einem der vorangehenden Ansprüche, wobei in Schritt (A) und/oder Schritt (B) mindestens eine Katalase zugegeben wird.

9. Verfahren nach einem der vorangehenden Ansprüche, wobei das künstliche Sputum zudem einen Wachstumszusatz für Mykobakterien umfasst.

10. Verfahren nach einem der vorangehenden Ansprüche, wobei der pH-Wert auf einen Wert im Bereich von 5,0 bis 8,0, bevorzugt im Bereich von 5,5 bis 7,5 eingestellt wird, bevor Schritt (B) durchgeführt wird.

11. Verfahren nach einem der vorangehenden Ansprüche, wobei die Menge an Mucin derart eingestellt wird, dass das künstliche Sputum eine Viskosität aufweist, die im Bereich von 75 bis 300 mPa·s, bevorzugt im Bereich von 100 bis 275 mPa·s liegt.

12. Künstliches Sputum, erhältlich durch das Verfahren nach einem der vorangehenden Ansprüche.

13. Verwendung von künstlichem Sputum nach Anspruch 12 in der Qualitätsbeurteilung oder zur Ausbildung in Bakteriologie.

14. Verwendung von künstlichem Sputum nach Anspruch 13, wobei die Bakterien aus einer Familie sind, die aus der Gruppe ausgewählt ist, welche aus Mycobacteriaceae besteht.

15. Verwendung von künstlichem Sputum nach Anspruch 13 oder 14, wobei die Qualitätsbeurteilung externe Qualitätsbeurteilung ist, bevorzugt eine externe Qualitätsbeurteilung von TB-Diagnostik durch Nachweis von *Mycobacterium-tuberculosis-Komplex*, bevorzugt in Ringversuchen, und/oder Qualitätsbeurteilung zur Bewertung und Prüfung von diagnostischen Tests und Geräten.

## Revendications

1. Procédé pour préparer une expectoration artificielle, comprenant les étapes suivantes :
une étape (A) consistant à préparer une solution ou dispersion aqueuse de nutriments, et
une étape (B) consistant à ajouter une mucine non humaine à la solution ou dispersion aqueuse obtenue dans l'étape (A) de sorte que la concentration de mucine se trouve dans la plage de 6 à 18 pour cent en poids, sur la base du poids total de l'expectoration artificielle, et que la viscosité de l'expectoration artificielle se trouve dans une plage de 30 à 480 mPa·s.

2. Procédé selon la revendication 1, dans lequel le procédé comprend en outre une étape de pasteurisation (C) consistant à pasteuriser la solution obtenue dans l'étape (B).

3. Procédé selon la revendication 2, dans lequel l'étape de pasteurisation (C) est exécutée en maintenant la solution à une température de 70 °C ou plus pendant une durée de 60 secondes ou plus.

4. Procédé selon l'une quelconque des revendications précédentes, dans lequel la solution aqueuse préparée dans l'étape (A) est stérilisée en autoclave à une température de 105 à 140 °C pour une durée de 1 minute à 24 heures à une pression de 1,1 à 5 bar avant l'exécution de l'étape (B).

5. Procédé selon l'une quelconque des revendications précédentes, dans lequel, après l'addition de mucine dans l'étape (B), la température est gardée en dessous de 90 °C.

6. Procédé selon l'une quelconque des revendications précédentes, dans lequel la quantité de mucine ajoutée dans l'étape (B) est telle que la concentration finale de mucine est dans la plage de 8 à 16 pour cent en poids, de préférence dans la plage de 10 à 14 pour cent en poids, sur la base du poids total de l'expectoration artificielle.

7. Procédé selon l'une quelconque des revendications précédentes, dans lequel la mucine non humaine est une mucine porcine ou une mucine bovine.

8. Procédé selon l'une quelconque des revendications précédentes, dans lequel au moins une catalase est ajoutée dans l'étape (A) et/ou dans l'étape (B).

9. Procédé selon l'une quelconque des revendications précédentes, dans lequel l'expectoration artificielle comprend en outre un supplément de croissance pour des mycobactéries.

10. Procédé selon l'une quelconque des revendications précédentes, dans lequel la valeur de pH est ajustée à une valeur dans la plage allant de 5,0 à 8,0, de préférence dans la plage allant de 5,5 à 7,5, avant l'exécution de l'étape (B).

11. Procédé selon l'une quelconque des revendications précédentes, dans lequel la quantité de mucine est ajustée de sorte que l'expectoration artificielle a une viscosité qui se trouve dans la plage allant de 75 à 300 mPa·s, de préférence dans la plage allant de 100 à 275 mPa·s.

12. Expectoration artificielle obtenue par le procédé selon l'une quelconque des revendications précédentes.

13. Utilisation d'une expectoration artificielle selon la revendication 12 dans une évaluation de qualité ou pour une formation en bactériologie.

14. Utilisation d'une expectoration artificielle selon la revendication 13, dans laquelle les bactéries sont issues d'une famille sélectionnée parmi le groupe constitué de mycobactériacées.

15. Utilisation d'une expectoration artificielle selon la revendication 13 ou 14, dans laquelle l'évaluation de qualité est une évaluation de qualité externe, de préférence une évaluation de qualité externe de diagnostics TB par détection du complexe Mycobacterium Tuberculosis, de préférence dans des essais circulaires et/ou une évaluation de qualité pour estimer et tester des tests ou dispositifs de diagnostic.
